# EUROPÄISCHE PATENTSCHRIFT

(11) **EP 3 385 367 B1**
(45) Veröffentlichungstag und Bekanntmachung des Hinweises auf die Patenterteilung: **04.05.2022**
(21) Anmeldenummer: 18165888.1
(22) Anmeldetag: 05.04.2018
(51) Int. Cl.: C12M 3/06, C12M 1/00, C12M 1/42, C12M 1/34

(54) **MIKROFLUIDISCHES SYSTEM ZUR KULTIVIERUNG VON ODER DER ANALYSE AN LEBENDEN ZELLEN ODER BIOMOLEKÜLEN SOWIE EIN VERFAHREN ZU SEINER HERSTELLUNG**
MICROFLUIDIC SYSTEM FOR CULTIVATING OR ANALYSING LIVING CELLS OR BIOMOLECULES, AND A METHOD FOR ITS MANUFACTURE
SYSTÈME MICROFLUIDIQUE DE CULTURE OU D'ANALYSE DE CELLULES VIVANTES OU DE BIOMOLÉCULES AINSI QU'UN PROCÉDÉ DE FABRICATION DUDIT SYSTÈME MICROFLUIDIQUE

(30) Priorität: 07.04.2017 DE 102017205978
(43) Veröffentlichungstag der Anmeldung: 10.10.2018
(73) Patentinhaber: Fraunhofer-Gesellschaft zur Förderung der angewandten Forschung e.V., 80686 München (DE)
(72) Erfinder: Busek, Mathias, 01277 Dresden (DE); Grünzner, Stefan, 01159 Dresden (DE); Sonntag, Frank, 01277 Dresden (DE); Partsch, Uwe, 01277 Dresden (DE); Feller, Claudia, 01277 Dresden (DE); Ihle, Martin, 01277 Dresden (DE)
(74) Vertreter: Pfenning, Meinig & Partner mbB

(56) Entgegenhaltungen:
- DE-A1-102007 046 305
- DE-A1-102010 038 445
- DE-A1-102012 102 021
- US-A1- 2005 106 066
- US-A1- 2006 154 361
- SAMUEL CHARLOT ET AL: "NOTE; A low cost and hybrid technology for integrating silicon sensors or actuators in polymer microfluidic systems", JOURNAL OF MICROMECHANICS & MICROENGINEERING, INSTITUTE OF PHYSICS PUBLISHING, BRISTOL, GB, Bd. 18, Nr. 1, 31. Januar 2008 (2008-01-31), Seite 17003, XP020129958, ISSN: 0960-1317
- ROSANNE M GUIJT ET AL: "Microfluidic chips for capillary electrophoresis with integrated electrodes for capacitively coupled conductivity detection based on printed circuit board technology", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 159, Nr. 1, 6. Juni 2011 (2011-06-06), Seiten 307-313, XP028280401, ISSN: 0925-4005, DOI: 10.1016/J.SNB.2011.06.023 [gefunden am 2011-06-12]

## Beschreibung

Die Erfindung betrifft ein mikrofluidisches System zur Kultivierung von oder der Analyse an lebenden Zellen oder Biomolekülen sowie ein Verfahren zu seiner Herstellung. Dabei können an lebenden Zellen unterschiedlichste Untersuchungen und Einflussmöglichkeiten durchgeführt und überwacht werden. So kann beispielsweise der Einfluss bestimmter Einflussgrößen bzw. Stoffe auf den Stoffwechsel von Zellen detektiert werden. Das System ist auch zur Untersuchung an verschiedenen Biomolekülen, wie z.B. Proteinen, Antikörpern, DNA oder RNA geeignet.

Für die Zellkultivierung werden aktuell vorrangig polymere Trägermaterialien verwendet. Dies liegt hauptsächlich an der guten Bioverträglichkeit, dem geringen Herstellungspreis (Spritzguss) und der guten optischen Zugänglichkeit begründet. Alle polymerbasierten Systeme haben allerdings das Problem, dass elektrochemische Sensoren z.B. zur Messung des Trans-Epithelialen-Widerstands (TEER) nur extern z.B. in Form von Chopstick-Elektroden eingebracht werden können. Die direkte Implementierung solcher Sensoren würde eine Reihe Vorteile mit sich bringen (Verringerung des Totvolumens, Verbesserung des Messsignals, Reduktion von Kontaminationen, ...). Hier bieten sich keramische Sensoren an, bei denen die elektrochemisch-aktiven Schichten mit einem Siebdruckprozess strukturiert aufgetragen und im Anschluss eingebrannt werden.

Die gleichzeitige Realisierung der fluidischen Strukturen in dem Keramiksubstrat, z.B. in LTCC-Technologie ausgeführt, besitzt den entscheidenden Nachteil, dass keramische Substrate optisch nicht transparent und darum für die Zellkultivierung und andere biotechnologische Anwendungen wenig geeignet sind. Überdies lassen sich in mehrlagigen Keramiksubstraten fluidische Aktoren nicht vergleichbar kostengünstig realisieren, wie dies z.B. durch die Integration von flexiblen Elastomermembranen in Polymerverbünden möglich ist.

Weiterhin sind hybride Multilagenaufbauten, wie mehrlagige Leiterplatten mit vergrabenen Bauelementen auf Si- oder Keramiksubstrat bekannt. Dabei werden Multilagenaufbauten auf Polymerträgern verwendet, die andere Materialien umschließen und elektrisch kontaktieren. Gängige Substrate sind Faserverbundwerkstoffe, wie FR-4 oder Polymerfolien, wie Polyimid, auf die Kupferfolie aufkaschiert wird. Diese Materialien besitzen keine oder nur bedingte Bioverträglichkeit oder sind nicht ausreichend transparent und sind darum für den Einsatz in der Zellkultivierung ungeeignet.

Daneben existieren bereits mikrofluidische Systeme, die auch für die Zellkultivierung genutzt werden und eine vergleichsweise hohe Integrationsdichte aufweisen und Sensoren und/oder Aktoren beinhalten. Dabei werden zumeist Glas-Silizium-Hybridsysteme verwendet, wobei sowohl die Strukturierung der mikrofluidischen Kanäle als auch die Herstellung der Sensoren bzw. Aktoren und deren elektrische Kontaktierung zumeist lithografisch erfolgt. Die strukturierten Lagen werden dann bevorzugt mittels anodischem Bonden zu einem fluiddichten Verbund gefügt. Nachteilig an dieser Herstellungstechnologie sind einerseits die hohen Kosten aufgrund der benötigten lithografischen Geräte, der teuren Substratmaterialien und der Masken sowie andererseits die niedrige Fertigungsausbeute, da viele aufwendige Herstellungsschritte nacheinander getätigt werden müssen.

Von S. Charlot u.a. sind in "A low cost and hybrid technology for integrating silicon sensors or actuators in polymer microfluidic systems"; J. Micromch. Microeng.; 18; 2008; S. 1-8 mikrofluidische Systeme mit gedruckten Schaltungsträgern (PCB) beschrieben. Eine ähnliche technische Lösung ist auf von Liang Li Wu u.a. in "Microfluidic printed circuit boards"; Electronic Components and Technology Conference (ECTC); 2011; IEEE 61st. IEEE 2011; S. 1576-1581 bekannt.

DE 10 2010 038 445 A1 betrifft ein Verfahren zur Herstellung eines mikrofluidischen Systems.

Eine mikrofluidische Vorrichtung zur Manipulation und Analyse von Fluiden ist in US 2005/0106066 A1 offenbart.

Es ist daher Aufgabe der Erfindung, Möglichkeiten für eine verbesserte Überwachung und Analyse an lebenden Zellen oder Biomolekülen anzugeben, und gleichzeitig eine gute Biokompatibilität, optische Zugänglichkeit sowie Flexibilität und Kosteneffizienz in der Herstellung einzuhalten.

Erfindungsgemäß wird diese Aufgabe mit einem mikrofluidischen System, das die Merkmale des Anspruchs 1 aufweist, gelöst. Ein Herstellungsverfahren für ein solches System ist mit dem Anspruch 9 definiert. Vorteilhafte Ausgestaltungen und Weiterbildungen der Erfindung können mit in untergeordneten Ansprüchen bezeichneten Merkmalen realisiert werden.

Bei dem System zur Kultivierung von oder die Analyse an lebenden Zellen oder Biomolekülen sind mehrere übereinander angeordnete Laminate aus einem polymeren Werkstoff fluiddicht und stoffschlüssig miteinander verbunden. Das dafür eingesetzte Polymer sollte bevorzugt optisch transparent sein.

Erfindungsgemäß ist in einem der Laminate mindestens eine Offnung oder mindestens ein Ausschnitt ausgebildet, mit der/dem ein Kanal oder ein Reservoir für die Aufnahme lebender Zellen oder Biomoleküle ausgebildet ist. Außerdem ist in mindestens einem der Laminate mindestens eine weitere Öffnung ausgebildet, in die ein aus einem keramischen oder einem halbleitenden Werkstoff gebildeter Aktor und/oder Sensor oder ein aus einem keramischen oder halbleitenden Werkstoff gebildeter Träger für einen Aktor oder Sensor eingesetzt ist. Ein Aktor und/oder Sensor sollte dabei so angeordnet und ausgebildet sein, dass ein Kontakt mit einer Flüssigkeit, in der lebende Zellen oder Biomoleküle enthalten sind, oder ein direkter Kontakt mit lebenden Zellen oder Biomolekülen möglich ist. Mindestens ein Aktor und/oder Sensor steht also in berührendem Kontakt mit einer Flüssigkeit für die Kultivierung von Zellen, einer Flüssigkeit in der Biomoleküle enthalten sind oder den in einem Kanal oder Reservoir enthaltenen Zellen oder Biomolekülen.

Auf der Oberfläche des Laminats in das der Aktor und/oder Sensor oder der Träger eingesetzt ist oder auf der Oberfläche eines unmittelbar an dieses Laminat angrenzenden Laminats ist mindestens eine elektrische Leiterbahn gedruckt, mit der eine elektrische Kontaktierung des Aktors und/oder Sensors erreichbar ist. Dies kann auch durch Überdrucken der Anschlusskontakte des Sensors/Aktors oder durch das mechanische Andrücken des selbigen an den gedruckten Leiterzug erfolgen.

Ein Aktor und/oder Sensor kann/können auf einer Oberfläche eines Trägers oder in einem Träger angeordnet sein. Als keramische Werkstoffe können beispielsweise LTCC, HTCC oder monolithische Materialien wie Aluminiumoxid, Zirkonoxid, Siliziumnitrid oder Aluminiumnitrid verwendet werden. Als halbleitenden Werkstoff kann Silizium in undotierter oder dotierter Form oder Siliziumdioxid eingesetzt werden.

Die Laminate sind erfindungsgemäß Folien und/oder plattenförmige Werkstücke. Die Laminate, welche ein System bilden, sollten möglichst aus dem gleichen Werkstoff bestehen oder zumindest aus sehr ähnlichen polymeren Werkstoffen bestehen, was insbesondere die Glasübergangstemperatur betrifft.

In mindestens einem der Laminate kann mindestens eine elektrische Durchkontaktierung, bei der in einer Öffnung ein elektrisch leitender Werkstoff enthalten ist, vorhanden sein. Es besteht aber auch die Möglichkeit, ein elastisch verformbares elektrisch leitendes Kontaktelement in eine solche Öffnung einzusetzen. Ein elastisch verformbares Kontaktelement kann aus einem Edelmetall, insbesondere Gold bestehen oder damit beschichtet sein.

Die Öffnungen sollten geometrisch so gestaltet und dimensioniert sein, dass ein Aktor und/oder Sensor bzw. ein Träger oder ein elastisch verformbares elektrisch leitendes Element formschlüssig mit einer entsprechenden Ausbildung an dessen Rand gehalten wird.

In einer vorteilhaften Ausführungsform kann zwischen zwei übereinander angeordneten Laminaten eine flexibel verformbare Leiterplatte angeordnet und fluiddicht mit den Laminaten verbunden sein. Die Leiterplatte kann entsprechend mit elektrischen und/oder elektronischen Bauelementen, die über elektrische Leiterbahnen miteinander verbunden sind, bestückt sein. Elektrisch leitende Verbindungen zu einem Aktor bzw. Sensor können über elektrische Durchkontaktierungen (Vias) oder mit elastisch verformbaren elektrisch leitenden Elementen erreicht werden.

Ein innerhalb des mit den Laminaten gebildeten Stapels angeordneter Sensor kann zur Erfassung physikalischer Größen z.B. Temperatur, Fluss, Volumenstrom, Druck, elektromagnetische Strahlung, elektrische Leitfähigkeit oder der stofflichen Zusammensetzung (z.B. des Sauerstoff-, Glukose- oder Laktatgehalts) genutzt werden oder ein Aktor sein. Aktoren können beispielsweise pneumatisch, thermopneumatisch, elektrostatisch, piezoelektrisch, elektromechanisch angetriebene Membranen oder Kolben sein. Aktoren können auch Elektroden zur elektrischen Zellstimulation, Elektroden zur Zell-/Partikel-Manipulation bzw. -Ablenkung mit Di/Elektrophorese, optoelektronische Komponenten oder piezoelektrische Aktoren sowie Temperierelemente zum Kühlen oder Heizen sein.

Im Bereich eines Kanals oder Reservoirs kann mindestens eine permeable Membran, bevorzugt eine gaspermeable Membran angeordnet sein. Damit erschließt sich die Möglichkeit gebildete Gase abzuführen oder eine Gaszufuhr vorzunehmen. So können beispielsweise stoffwechselbedingt gebildete Gase abgeführt und Sauerstoff für die Nährstoffversorgung zugeführt werden. Diese können dann insbesondere was ihre Zusammensetzung und die jeweiligen Volumina betrifft detektiert und ein ggf. aufgetretener Einfluss auf den Stoffwechsel erkannt und nachgeregelt werden. Gaspermeable Membranen können beispielsweise aus Silikonfolien, dünnen Thermoplasten oder thermoplastischen Elastomeren (TPE) gebildet werden. Poröse Membranen (z.B. laserstrukturiere oder track-etched PC-Folie) können z.B. als Zellträger oder zur Herstellung elektrochemischer Sensoren in ein erfindungsgemäßes System integriert sein.

Ein Kanal oder Reservoir kann mit einer Zu- und/oder Abführung für eine Flüssigkeit, welche lebende Zellen und/oder Biomoleküle enthalten kann, verbunden sein. So kann für eine Zu- oder Abführung jeweils ein Anschlussstutzen durch eine nach außen geführte Öffnung, die in mindestens einem Laminat ausgebildet ist, eingeführt und mit dem jeweiligen Laminat eine fluiddichte und stoffschlüssige Verbindung ausgebildet werden.

Es besteht auch die Möglichkeit, dass auf mindestens einer Oberfläche mindestens eines Laminats elektrische und/oder elektronische Elemente, insbesondere ein elektrischer Widerstand, ein induktives elektrisches Element, ein kapazitives elektrisches Element oder ein Transistor mittels eines Druckverfahrens ausgebildet sind. Sensorische Elemente, wie z.B. Dehnmessstreifen für die Druckmessung oder Elektrodenstrukturen, z.B. für Partikel- oder Zellmanipulation können mit diesem Verfahren ebenfalls direkt gedruckt werden.

Ein Kanal oder ein Reservoir kann mit einer semipermeablen Membran an einem Laminat zumindest bereichsweise abgedeckt sein. Die Membran kann dabei für ein Gas permeabel und ggf. für eine Flüssigkeit, wie z.B. einen Elektrolyten impermeabel sein. So kann beispielsweise Sauerstoff oder Kohlendioxid durch die Membran permeieren und in einem auf der anderen Seite der Membran, an der beispielsweise keine Zellkulturen in einer Öffnung vorhanden sind, einem weiteren dort angeordneten Kanal oder Reservoir aber ein geeigneter die jeweilige Gaskonzentration bestimmender Sensor angeordnet sein, mit dem Untersuchungen des Stoffwechsels von Zellkulturen durchgeführt werden können.

Ein Kanal oder ein Reservoir kann aber auch mit einer elastisch verformbaren Membran zumindest bereichsweise abgedeckt sein, wobei die Membran mit elektrisch leitenden gedruckten Strukturen, insbesondere Dehnmessstreifen bedruckt sein kann. Damit ist beispielsweise eine Druckmessung im System möglich.

Ein oder mehrere Membran(en) kann/können beim Fügen der Laminate zwischen benachbarten Laminaten einlaminiert und fluiddicht mit den jeweiligen die Membranoberfläche berührenden Oberflächen der Laminate stoffschlüssig am äußeren Rand der jeweiligen Membran verbunden werden.

Bei der Herstellung eines erfindungsgemäßen Systems wird so vorgegangen, dass in mindestens einem Laminat, das zwischen einem oberhalb und unterhalb anzuordnenden Laminat angeordnet werden soll, mindestens eine Öffnung oder Aussparung zur Ausbildung eines Kanals oder Reservoirs und in mindestens einem oberhalb oder unterhalb dieses Laminats anzuordnenden Laminats mindestens eine weitere Öffnung zur Aufnahme eines aus keramischen oder halbleitenden Werkstoff gebildeten Aktors und/oder Sensors oder einen Träger eines Aktors und/oder Sensors, der aus einem keramischen oder halbleitenden Werkstoff gebildet ist, ausgebildet werden.

In die mindestens eine weitere Öffnung wird ein Aktor und/oder Sensor oder ein Träger eingesetzt. Auf eine Oberfläche dieses Laminats oder eines unmittelbar benachbart anzuordnenden Laminats wird mindestens eine elektrische Leiterbahn für eine elektrische Kontaktierung des Aktors und/oder Sensors mit einem Druckverfahren ausgebildet.

Die Laminate werden dann so übereinander angeordnet, dass die mindestens eine einen Kanal oder ein Reservoir bildende Öffnung und der mindestens eine Aktor und/oder Sensor zueinander positioniert sind und die den Stapel bildenden Laminate durch eine thermische Behandlung und bevorzugt gleichzeitig wirkenden Druckkräften fluiddicht und stoffschlüssig miteinander verbunden werden.

Die Öffnungen können mittels Stanzen, Prägen, mechanischem Materialabtrag, Laserstrahlschneiden und -ablation oder lithografisch ausgebildet werden.

Mit den Öffnungen oder Aussparungen können Kanäle oder Reservoire mit Abmessungen im Bereich von 10 µm bis 5 mm, was insbesondere deren Höhe und Breite betrifft ausgebildet werden. Ein Kanal kann eine deutlich größere Länge haben und auch nicht ausschließlich entlang einer geradlinigen Längsachse ausgerichtet sein, sondern auch zumindest bereichsweise bogenförmig ausgebildet sein.

Das Aufdrucken elektrischer Leiterbahnen und ggf. weiterer elektrischer und/oder elektronischer Bauelemente kann mit direktschreibenden Verfahren (Aerosol-Jet oder Ink-Jet-Druck) von nanopartikelhaltigen Tinten oder mit Siebdruck (polymere Pasten/nanopartikelhaltigen Pasten) erreicht werden. Es können auch gängige Verbindungstechniken, wie z.B. Löten und/oder elastisch verformbare Kontaktelemente zur Herstellung eines elektrischen Kontakts mit peripherer Elektronik verwendet werden.

Für das Drucken und auch für die Herstellung elektrisch leitender Durchkontaktierungen können an sich bekannte Pasten oder Suspensionen eingesetzt werden. Diese enthalten elektrisch leitende Partikel, die bei einer thermischen Behandlung miteinander zumindest teilweise versintert werden können. Flüssige und polymere Bestandteile können dabei durch Verdampfen oder durch Sublimation entfernt werden.

Mindestens ein Kanal oder mindestens ein Reservoir kann mit Öffnungen oder Aussparungen, die in mehreren benachbart zueinander im Laminatstapel angeordneten Laminaten ausgebildet sind, ausgebildet sein. So können auch dreidimensionale Fluidsysteme über mehrere Lagen ausgebildet werden.

Fluidische Strukturen in Form von Kanälen und/oder Reservoiren können nicht nur in Laminaten sondern auch auf einem keramischen oder halbleitenden Werkstoff, wie dies beispielsweise ein Träger sein kann, ausgebildet sein. Diese Strukturen können mit Kanälen und/oder einem Reservoir, die/das im Laminatstapel ausgebildet ist/sind, verbunden sein.

Mit fluidischen Strukturen können beispielsweise Mikromischer oder Partikelsortierer realisiert werden.

Aussparungen können bis an den äußeren Rand geführt sein, so dass von dort aus eine Zu- und Abfuhr von Flüssigkeit möglich ist.

Für die Laminate können beispielsweise Polycarbonat (PC), Cyclo-Olefine-Copolymer (COC), Polyethylen (PE), Polyethylenterephthalat (PET), Polypropylen (PP), Polystyrol (PS), Polyimid (PI), Polymethylmethacrylat (PMMA), Polyetheretherketon (PEEK), Polytetrafluorethylen (PTFE) oder Silikone, wie z.B. Polydimethylsiloxan (PDMS) und andere Polyorganosiloxane als polymerer Werkstoff eingesetzt werden.

Als Fügetechnologie können thermisches Diffusionsbonden, Plasmafügen, nasschemische Verfahren, laserunterstütztes Fügen, Ultraschallfügen und/oder deren Kombination genutzt werden. Sensoren/Aktoren sollten dabei direkt in den Verbund fluidisch dicht integriert werden können.

Bei der Erfindung können polymere Basismaterialien verwendet werden, die jeweils einzeln strukturiert, gefügt und bedruckt werden können. Das resultierende System ist weitaus kostengünstiger als Glas-Silizium-basierte Systeme und erlaubt überdies die einfache Implementierung fluidischer Aktoren, wie z.B. Pumpen und Sensoren, wie z.B. Flusssensoren. Mit der beschriebenen Herstellungstechnologie können beliebig viele weitere (auch kommerziell erhältliche) Sensoren/Aktoren auf oder mit einem Keramik- oder Halbleiterwerkstoff erweitert werden. Durch eine veränderte Ausbildung der Laminate mit unterschiedlicher Anordnung und Dimensionierung von Öffnungen können auf einfache Weise unterschiedlich konfigurierte Systeme für verschiedenste Anwendung in entsprechend angepasster Form zur Verfügung gestellt werden.

Es können mikrofluidische Systeme realisiert werden, bei denen mehrlagenkeramische Werkstoffe (LTCC) mit polymeren Werkstoffen (PDMS) über einen Plasma-Aktivierungsprozess der Oberflächen gefügt werden.

Mikrofluidische Kanäle und sensorische und/oder aktorisch wirkende Elemente können zusätzlich auch innerhalb des keramischen Werkstoffs realisiert werden. Mehrlagenkeramische Sensoren können innerhalb des jeweiligen polymeren Werkstoffs, mit dem die Laminate gebildet sind, eingebettet und elektrisch kontaktiert werden. Der polymere Werkstoff kann die mikrofluidischen, insbesondere die strömungsmechanischen Funktionen übernehmen.

Um die Vorteile beider Technologien zu vereinigen, werden keramische Sensoren und/oder Aktoren in polymere, mikrofluidische Multilagensysteme integriert. Dabei kann die optische Zugänglichkeit von außen weitgehend erhalten werden, weil die Sensoren/Aktoren vergleichsweise klein ausgelegt werden können und z.B. mit direktschreibenden Druckverfahren mit niedrigtemperatursinternden nanopartikelhaltigen Tinten elektrisch kontaktierbar bleiben. Dadurch lassen sich mikrofluidische Systeme herstellen, die durch die direkte Implementierung insbesondere elektrischer Sensoren/Aktoren oder auch von optoelektronischen Komponenten eine größere Integrationsdichte und einen größeren Funktionsumfang aufweisen.

Die Erfindung ermöglicht die Integration verschiedener Sensoren und/oder Aktoren (z.B. elektrochemische Sensoren für die pH-Messung, Elektroden oder Anregungs-LEDs).

Folgende Fertigungsschritte sollten bei der Herstellung nacheinander durchgeführt werden:
1. Herstellung einer Halbschale für die Sensor-/Aktorintegration aus strukturierten Polymerfolien oder -platten, wobei in mindestens einem Laminat Öffnungen zum Einlegen von Aktoren bzw. Sensoren, der Ausbildung mindestens eines Kanals oder Reservoirs vorgesehen sind. Die Strukturierung der Folien kann mechanisch z.B. durch Mikrofräsen, Stanzen, Prägen, oder mittels elektromagnetischer Strahlung bevorzugt mittels Laserablation erfolgen.
2. Einlegen der Sensoren/Aktoren in dafür vorgesehene Öffnungen.
3. Siegeln und ggf. Abdichten der Sensoren/Aktoren durch z.B. Einkleben, Einlaminieren, Pressen, chemisches Bonden, Ultraschallfügen oder thermisches Bonden und/oder Kombinationen davon.
4. Elektrische Kontaktierung der Sensoren/Aktoren mit direktschreibenden Verfahren (Aerosol-Jet oder Ink-Jet-Druck von nanopartikelhaltigen Tinten), Siebdruck (polymere Pasten/ nanopartikelhaltige Pasten)
5. Erneutes Fügen/Siegeln der bedruckten Polymerfolien als Laminate mit weiteren strukturierten Polymerfolien oder -platten, um vergrabene elektrische Leiterbahnen herzustellen.
6. Durch das Verfüllen von nach oben offenen bzw. vergrabenen Öffnungen, bevorzugt in Form von Löchern, mit elektrisch leitfähigen polymeren Pasten/ nanopartikelhaltigen Pasten können elektrische Durchkontaktierungen generiert werden.
7. Die elektrische Kontaktierung mit peripherer Elektronik, z.B. für die Sensor- Signalkonditionierung/Signalverarbeitung, kann direkt durch den Druck kontaktierbarer Anschlusskontakte (Pads), über elastisch verformbare elektrische Kontaktelemente aus einem Edelmetall, insbesondere Gold oder durch die Integration einer lötfähigen FLEX-Leiterplatte (analog zur Integration der Sensoren/Aktoren) erfolgen.
8. Zur Herstellung fluidischer und/oder pneumatischer Anschlüsse können verschiedene Konnektoren durch Kleben, Pressen, chemisches Bonden, Laserfügen, Ultraschallfügen oder thermisches Bonden und/oder Kombinationen davon aufgebracht werden und das System in einer Halterung mit pneumatischen und/oder fluidschen Anschlüssen fixiert werden.

Der klare Vorteil der beschriebenen Technologie liegt in der Möglichkeit die Vorteile der einzelnen unterschiedlichen Teiltechnologien zu bündeln und dabei die jeweiligen einzelnen Nachteile zu kompensieren. Im Gegensatz zu reinen Multilagen-Keramikaufbauten ist die erfindungsgemäße Hybridtechnologie weitaus kostengünstiger und ressourcenschonender. Es lassen sich mit dieser Technologie Funktionen realisieren, die in polymeren Mikrosystemen ohne weiteres nicht direkt umsetzbar sind. Ein Beispiel dafür sind elektrochemische Sensoren, da für diese keine entsprechenden nanopartikelhaltigen Tinten für den direkten Druck verfügbar sind. Da die optische Zugänglichkeit durch den Einsatz transparenter Folien- oder Plattensubstrate und miniaturisierter Sensoren nur minimal erschwert wird, können die kultivierten Zellen gut beobachtet oder der Zustand oder die Anzahl von Biomolekülen beurteilt bzw. bestimmt und optische Funktionen einfach realisiert werden. So lassen sich zum Beispiel auch auf kleinem Bauraum optische Linsen und andere optische Komponenten mit Detektoren und Anregungs-LEDs o.Ä. kombinieren. Der direkte Druck von Sensoren und Aktoren aber auch von passiven elektrischen Bauelementen, wie elektrischen Widerständen oder Kondensatoren ist mit der Erfindung realisierbar und ermöglicht zukünftig eine Vorverarbeitung der Sensorsignale z.B. zur Temperaturkompensation. Weiterhin bietet die modulare Herstellungstechnologie den Vorteil, dass die einzelnen Komponenten (Sensoren, Aktoren, Substrat mit mikrofluidischen Strukturen) unabhängig voneinander gefertigt und getestet werden können, bevor diese zusammengefügt werden. Dadurch verringert sich die Gesamtausfallswahrscheinlichkeit des Systems und es kann eine Art Baukasten zur Herstellung komplexer mikrofluidischer Systeme zur Verfügung gestellt werden. Elektrische Leiterbahnen können so ausgebildet und betrieben, werden, dass eine elektrische Widerstandsheizung, möglichst in geregelter Form realisiert werden kann. Auch können sensorische und/oder aktorische Elemente direkt gedruckt werden.

Neben dem zuvor beschriebenen Anwendungsfall bei der Kultivierung humaner Zellen bzw. dem Überwachen der Kultivierungsparameter (Sauerstoff, Glucose, Temperatur, Zellvitalität...) sowie zur Stimulation der Zellen (elektrisch, mechanisch, optisch) gibt es eine Reihe weiterer Anwendungsmöglichkeiten in der Biotechnologie. Diese sind zum Beispiel:
- Die Sortierung von Zellen z.B. mit elektrischen oder magnetischen Feldern,
- der Nachweis oder die Synthese von Biomolekülen (RNA, Antikörper),
- die Einzelzellmanipulation (Fusionierung, Injektion, ...),
- die Umweltanalytik z.B. in Form von Ganzzellsensoren,
- oder spezifische Fragestellungen, wie z.B. on-Chip PCR oder DNA-Hybridisierung.

Nachfolgend soll die Erfindung beispielhaft näher erläutert werden.

Dabei zeigen:
Figur 1 mehrere Laminate in denen jeweils mehrere Öffnungen ausgebildet sind und die zu einem Stapel, bei dem die Laminate übereinander angeordnet sind, mit einer Heißpresse 9 gefügt werden können;
Figur 2 eines der in Figur 1 gezeigten Laminate mit Öffnungen in die jeweils ein Sensor eingesetzt wird;
Figur 3 das in Figur 2 gezeigte Laminat, bei dem elektrische Leiterbahnen als elektrisch leitende Verbindung zu den Sensoren aufgedruckt wurden;
Figur 4 zwei Laminate, wobei der untere den eingelegten, bedruckten Sensor enthält und das obere Laminat Vias zur elektrischen Kontaktierung beinhaltet und durch Fügen in der Heißpresse ein Laminat mit vergrabenem elektrisch kontaktierten Sensor entsteht;
Figur 5 die Endmontage des Systems durch Laminieren aller verbliebenen Laminate und dem Aufbringen von fluidischen Anschlüssen;
Figur 6 ein weiteres Ausführungsbeispiel eines elektrochemischen Sensors mit Elektrolytraum abgetrennt durch eine Membran und eingebrachten Elektroden auf Keramiksubstrat und
Figur 7 weiteres Ausführungsbeispiel eines auf eine flexible Membran gedruckten Drucksensors.

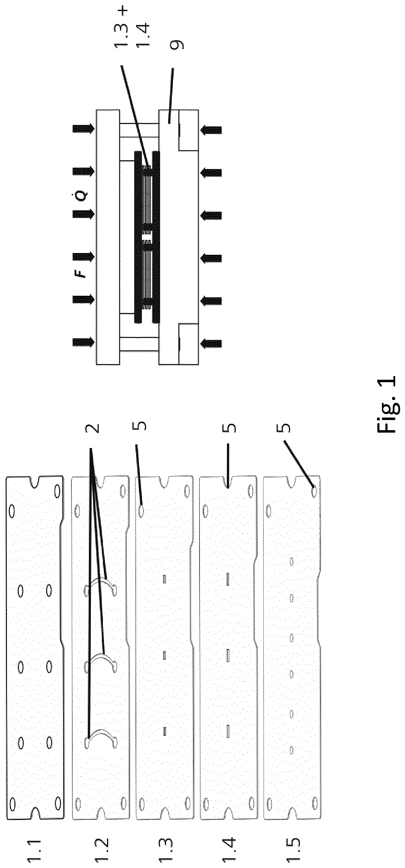

Die in Figur 1 gezeigten Laminate 1.1, 1.2,....1.5) aus Polycarbonat haben eine jeweils konstante Schichtdicke von 0,25 mm. Durch Laserablation wurden in den Laminaten Öffnungen ausgebildet. Die Öffnungen im Laminat 1.2, das im gefügten Stapel das zweite Laminat von oben bildet, sind die bogenförmigen Öffnungen als Kanäle 2 ausgebildet, in denen Zellen kultiviert oder Biomoleküle untersucht werden können.

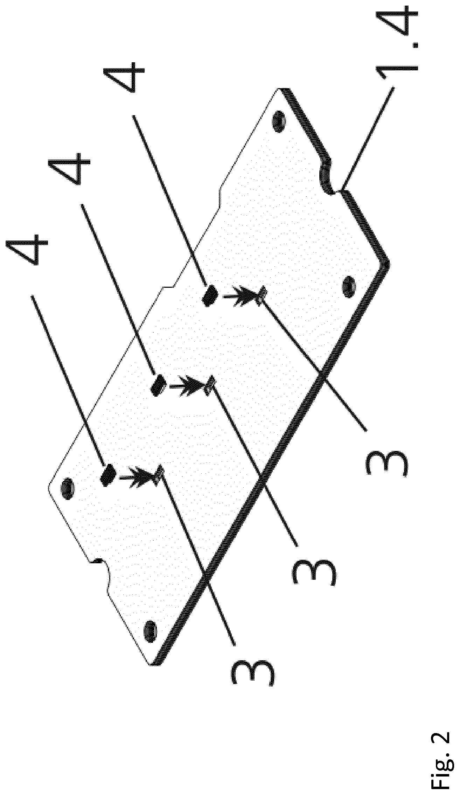

In dem darunter anzuordnenden Laminaten 1.3 und 1.4 sind Öffnungen 3 ausgebildet, in die jeweils ein einem der Kanäle 2 zugeordneter Sensor 4 einsetzbar ist, wie dies auch in Figur 2 gezeigt ist. Die Einbettung der Sensoren 4 kann auf verschiedene Weise erfolgen:
- Formschlüssige Einpassung des Sensors 4 durch Einbringung einer verformbaren Opferschicht in den Spalt zwischen Sensor 4 und Laminat (z.B. aus einem Thermoplast mit anderen Fließ- und Erweichungseigenschaften)
- Übermaßpassung und Ausnutzung der größeren Härte des einzubringenden Sensorsubstrats.
- Einkleben des Sensors in eine Passung mit Spiel.

In analoger Form können natürlich auch Aktoren oder Kombinationen von Sensoren mit Aktoren in eine Öffnung eingesetzt und ggf. darin fixiert werden.

Mit weiteren in den anderen Laminaten z.B. 1.5 ausgebildeten Öffnungen können elektrische Durchkontaktierungen 10 (Vias) ausgebildet werden.

Die Aussparungen 5 an den Stirnseiten, sowie die Positionierungslöcher 5 der Laminate 1.1 bis 1.5 können für eine Justierung und Positionierung vor und während des Fügens des Systems genutzt werden.

Zunächst werden die Sensoren 4 in die dafür vorgesehenen Öffnungen der Laminate 1.3 und 1.4 eingesetzt. Im Anschluss daran werden elektrische Leiterbahnen 6 auf die Oberfläche des Laminats 1.4 so aufgedruckt, dass eine elektrisch leitende Verbindung der Sensoren 4 nach außen oder zu einer hier nicht gezeigten ggf. aber im Laminatstapel integrierten flexibel verformbaren Leiterplatte über elektrische Durchkontaktierungen (Vias) 10 erreichbar ist. Die elektrischen Durchkontaktierungen 10 können mit einem elektrisch leitenden Werkstoff ausgefüllt oder an den Innenwänden elektrisch leitend beschichteten Öffnungen, die ebenfalls in den Laminaten ausgebildet wurden, hergestellt werden.

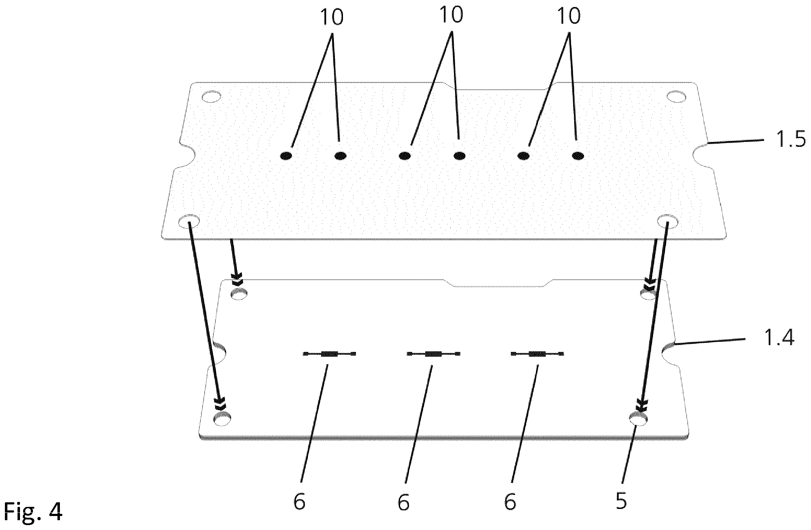

In Figur 4 ist der Prozess der Sensor-Einbettung und der Herstellung von Vias 10 gezeigt. Durch Fügen der Laminate 1.4 und 1.5 entsteht eine elektrisch kontaktierte Sensorstruktur mit Vias 10, die nach unten hin ausgeführt sind um den jeweiligen Sensor 4 von der Unterseite z.B. über elastisch verformbare elektrisch leitende Kontaktelemente zu kontaktieren. Der Sensor 4 selbst ist zwischen den mit Laminaten gebildeten Lagen eingebettet und steht in direktem Kontakt zum Kanal 2 und der darin geführten Flüssigkeit. Die gedruckten elektrischen Ankontaktierungen 6 des Sensors 4 werden bei Temperaturen knapp unterhalb der Glasübergangstemperatur der Laminate (1.1, ....1.5) unter Druck in der Heißpresse 9 ausgesintert, um eine verbesserte elektrische Leitfähigkeit zu erreichen.

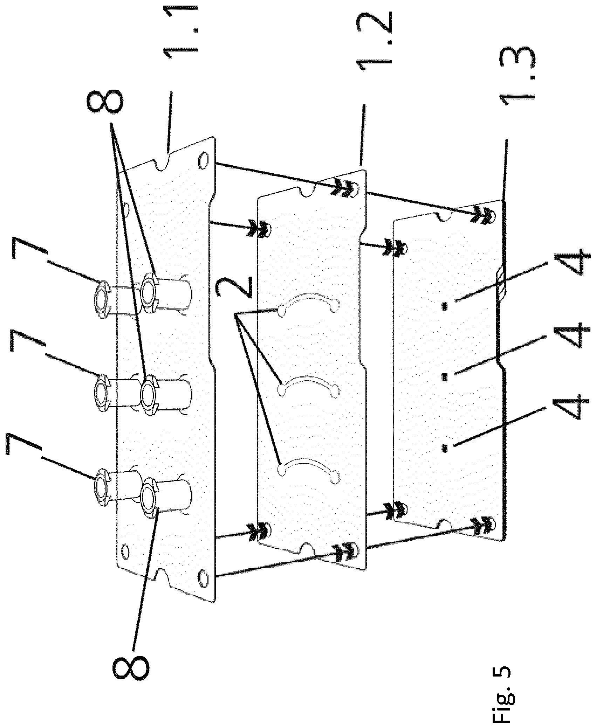

Bei dem in Figur 5 oben gezeigten Laminat 1.1 sind Öffnungen für jeweils einen Zufluss 7 und einen Abfluss 8 vorhanden. Über den Zufluss 7 kann eine Flüssigkeit, bei der es sich um eine zur Kultivierung von lebenden Zellen geeigneten Flüssigkeit, die ggf. einen oder mehrere Wirkstoff(e) enthalten kann und/oder eine Flüssigkeit in der lebende Zellen oder Biomoleküle enthalten sind handeln kann, eingebracht werden.

Die Zu- und Abflüsse 7 und 8 sind mit dem Laminat 1.1 fluiddicht und stoffschlüssig verbunden und so angeordnet, dass sie mit jeweils einem stirnseitigen Ende eines Kanals 2, der im Laminat 1.2 ausgebildet ist, in Kontakt stehen.

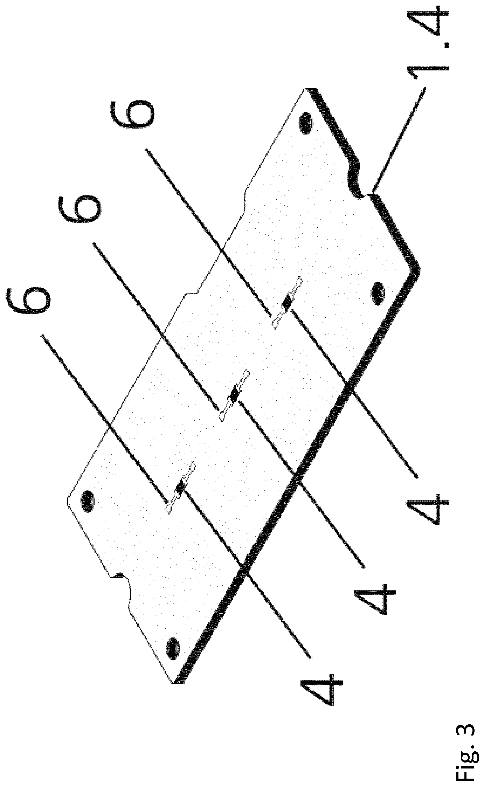

Im Laminat 1.3 sind Öffnungen 3 ausgebildet in die hier nicht gezeigte Sensoren 4 einsetzbar sind, wie dies aber in den Figuren 2 und 3 gezeigt ist.

Nach dem die Laminate 1.1 bis 1.5 unten und oben des mit den Laminaten 1.2, 1.3 und 1.4 gebildeten Stapels angeordnet und zueinander ausgerichtet sind, wird der so erhaltene Stapel in eine Heißpresse 9 eingesetzt. Je nachdem erfolgt das Fügen auch in mehreren Schritten, um zum Beispiel die in Figur 4 gezeigten vergrabenen Sensorstrukturen zu generieren. Dabei erfolgte eine thermische Behandlung bei der die Laminate bei einer maximalen Temperatur von 70 °C - 200 °C stoffschlüssig miteinander verbunden werden. Dabei wird gewährleistet, dass die in den Kanälen 2 enthaltene Flüssigkeit außer aus Zu-oder Abfluss 7 oder 8 nicht entweichen kann.

Während der thermischen Behandlung werden auf den Stapel Druckkräfte mit einem maximalen Druck von 13 MPa ausgeübt.

Während der thermischen Behandlung erfolgt eine zumindest teilweise Sinterung der Nanopartikel mit denen elektrische Leiterbahnen 6 und ggf. elektrische Durchkontaktierungen 10 ausgebildet wurden. Während der Lamination des Folienstapels wird die Sinterung der Nanopartikel durch die erfolgte Druckbeaufschlagung verstärkt. Die Partikel können beispielsweise aus Silber, Gold, Platin, Kupfer oder Legierungen davon bestehen.

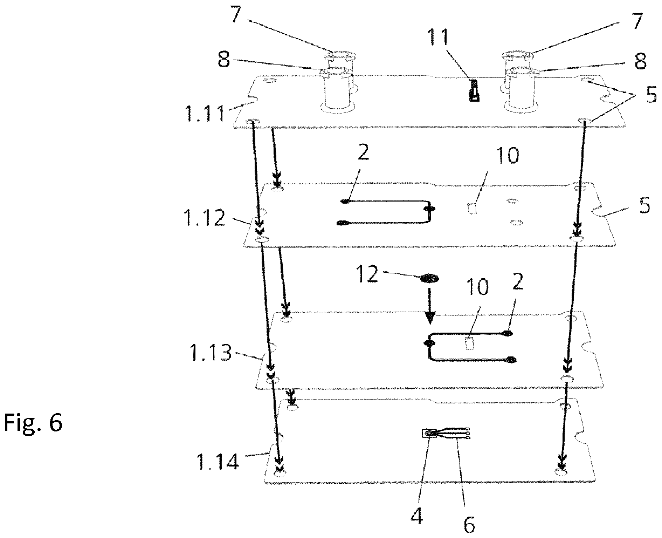

In Figur 6 ist als weiteres Ausführungsbeispiel ein elektrochemischer Sensor, z.B. zur Messung des Sauerstoffgehalts dargestellt. Dieser besteht aus 4 Laminaten 1.10 - 1.14, wobei die unterste Lage 1.14 den elektrochemischen Sensor 4 enthält, der analog zu Figur 3 durch aufgedruckte elektrische Leiterbahnen 5 elektrisch kontaktiert wird. Darüber befindet sich in Laminat 1.13 ein eingebrachter Mikrokanal 2, in dem sich ein Elektrolyt befindet. Der Elektrolyt ist vom jeweiligen Messgut im Laminat 1.12 durch eine eingebrachte Membran 12 getrennt. Diese Membran 12 ist durchlässig für Sauerstoff aber impermeabel für den Elektrolyten und wird direkt in den Verbund mit eingefügt. Die Laminate 1.11 - 1.13 enthalten Ausbrüche für die elektrische Kontaktierung des Sensors 4, der mittels einer Stiftleiste 11 im obersten Laminat 1.11 mit der peripheren Elektronik verbunden werden kann. Über die Fluideinlässe 7 und -Auslässe 8 kann sowohl der Elektrolyt als auch das Messgut (z.B. Kulturmedium) gewechselt bzw. gezielt zugeführt werden.

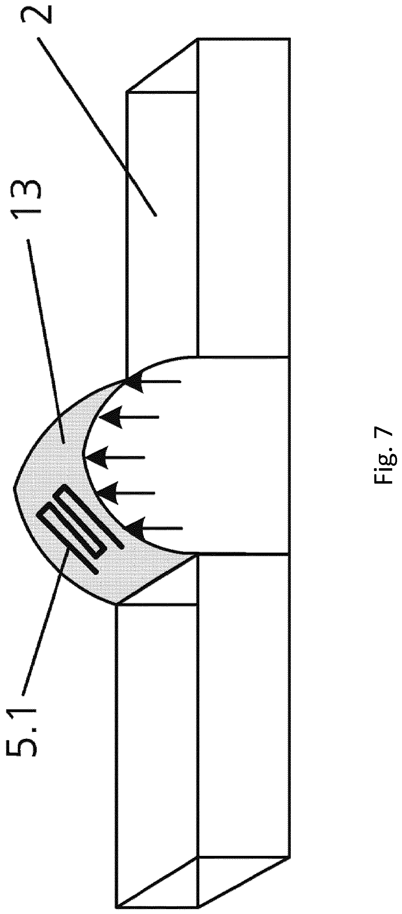

Schließlich zeigt Figur 7 eine weitere Ausführung der Erfindung in Form eines Drucksensors. Dabei ist der Mikrokanal 2 durch eine flexible Membran 13 nach oben hin abgeschlossen, auf die gedruckte Strukturen 5.1 aufgebracht sind. Diese bevorzugt elektrisch leitenden gedruckten Strukturen 5.1 können durch Wahl einer piezoresistiven Tinte zur Realisierung eines Dehnmessstreifens und damit zur Messung der Membranauslenkung bzw. des Drucks im Kanal 2 genutzt werden.

## Patentansprüche

1. Mikrofluidisches System zur Kultivierung von oder der Analyse an lebenden Zellen oder Biomolekülen, bei dem mehrere übereinander angeordnete Laminate (1.1, 1.2, ...1.x) aus einem polymeren Werkstoff, die als Folien und/oder plattenförmige Werkstücke ausgebildet sind, fluiddicht und stoffschlüssig miteinander verbunden und in mindestens einem der Laminate (1.1, 1.2, ...1.x) mindestens eine Öffnung oder mindestens ein Ausschnitt ausgebildet ist, mit der/dem ein Kanal (2) oder eine Reservoir für die Aufnahme lebender Zellen oder Biomoleküle ausgebildet ist, und
in mindestens einem der Laminate (1.1, 1.2, ...1.x) mindestens eine weitere Öffnung (3) ausgebildet ist, in die ein aus einem keramischen oder einem halbleitenden Werkstoff gebildeter Aktor und/oder Sensor (4) oder ein aus einem keramischen oder halbleitenden Werkstoff gebildeter Träger für einen Aktor oder Sensor eingesetzt ist, und auf der Oberfläche des Laminats (1.2) in das der Aktor und/oder Sensor (4) oder der Träger eingesetzt ist oder auf der Oberfläche eines unmittelbar an dieses Laminat (1.2) angrenzenden Laminats (1.3) mindestens eine elektrische Leiterbahn (6) gedruckt ist, mit der eine elektrische Kontaktierung des Aktors und/oder Sensors (4) erreichbar ist; wobei der mindestens eine Aktor und/oder Sensor (4) in berührendem Kontakt mit einer Flüssigkeit für die Kultivierung von Zellen, einer Flüssigkeit in der Biomoleküle enthalten sind oder den in einem Kanal oder Reservoir enthaltenen Zellen oder Biomolekülen steht.

2. System nach Anspruch 1, **dadurch gekennzeichnet, dass** in mindestens einem der Laminate (1.1, 1.2, ...1.x) mindestens eine elektrische Durchkontaktierung, bei der in einer Öffnung ein elektrisch leitender Werkstoff enthalten ist und/oder ein elastisch verformbares elektrisch leitendes Kontaktelement eingesetzt ist.

3. System nach einem der vorhergehenden Ansprüche, **dadurch gekennzeichnet, dass** zwischen zwei übereinander angeordneten Laminaten eine flexibel verformbare Leiterplatte angeordnet und fluiddicht mit den Laminaten verbunden ist.

4. System nach einem der vorhergehenden Ansprüche, **dadurch gekennzeichnet, dass** ein innerhalb des mit den Laminaten (1.1, 1.2, ...1.x) gebildeten Stapels angeordneter Sensor (4) zur Erfassung physikalischer Größen, insbesondere der Temperatur, des Flusses, des Volumenstromes, des Druckes, elektromagnetischer Strahlung, der elektrischen Leitfähigkeit oder der stofflichen Zusammensetzung, insbesondere des Sauerstoffgehalts, Glukosegehalts, Laktatgehalts und/oder ein Aktor, insbesondere eine Flussquelle, ein elektromagnetische Strahlung emittierendes Element, eine Elektrode, eine aktiv verformbare Membran, ein Piezo-Aktor, eine gasduchlässige Membran, ein Temperierelement angeordnet ist.

5. System nach einem der vorhergehenden Ansprüche, **dadurch gekennzeichnet, dass** im Bereich eines Kanals (2) oder Reservoirs mindestens eine permeable Membran, bevorzugt eine gaspermeable Membran angeordnet ist.

6. System nach einem der vorhergehenden Ansprüche, **dadurch gekennzeichnet, dass** ein Kanal (2) oder Reservoir mit einer Zu- und/oder Abführung für eine lebende Zellen und/oder Biomoleküle enthaltende Flüssigkeit verbunden ist.

7. System nach einem der vorhergehenden Ansprüche, **dadurch gekennzeichnet, dass** auf mindestens einer Oberfläche mindestens eines Laminats elektrische und/oder elektronische Elemente, insbesondere ein elektrischer Widerstand, ein Thermoelement, ein induktives elektrisches Element, ein kapazitives elektrisches Element oder aktive Komponenten, wie ein Transistor mittels eines Druckverfahrens ausgebildet ist.

8. System nach einem der vorhergehenden Ansprüche, **dadurch gekennzeichnet, dass** ein Kanal (2) oder ein Reservoir mit einer semipermeablen Membran (12) an einem Laminat (1.12) zumindest bereichsweise abgedeckt ist
oder
ein Kanal (2) oder ein Reservoir mit einer elastisch verformbaren Membran (13) zumindest bereichsweise abgedeckt und die Membran (13) mit elektrisch leitenden gedruckten Strukturen 5.1, insbesondere Dehnmessstreifen bedruckt ist.

9. Verfahren zur Herstellung eines Systems nach einem der vorhergehenden Ansprüche, **dadurch gekennzeichnet, dass** in mindestens einem Laminat, das zwischen einem oberhalb und unterhalb anzuordnenden Laminat angeordnet werden soll, mindestens eine Öffnung oder Aussparung zur Ausbildung eines Kanals (2) oder Reservoirs und
in mindestens einem oberhalb oder unterhalb dieses Laminats anzuordnenden Laminats mindestens eine weitere Öffnung zur Aufnahme eines aus keramischen oder halbleitenden Werkstoff gebildeten Aktors und/oder Sensors (4) oder einen Träger eines Aktors und/oder Sensors, der aus einem keramischen oder halbleitenden Werkstoff gebildet ist, ausgebildet werden und
in die mindestens eine weitere Öffnung ein Aktor und/oder Sensor (4) oder ein Träger eingesetzt und
auf eine Oberfläche dieses Laminats oder eines unmittelbar benachbart anzuordnenden Laminat mindestens eine elektrische Leiterbahn (6) für eine elektrische Kontaktierung des Aktors und/oder Sensors (4) mit einem Druckverfahren ausgebildet wird und
die Laminate so übereinander gestapelt angeordnet werden, dass die mindestens eine einen Kanal (2) oder ein Reservoir bildende Öffnung und der mindestens eine Aktor und/oder Sensor (4) zueinander positioniert sind und
die den Stapel bildenden Laminate durch eine thermische Behandlung und gleichzeitig wirkenden Druckkräften fluiddicht und stoffschlüssig miteinander verbunden werden; wobei Laminate (1.1, 1.2, ...1.x) eingesetzt werden, die als Folien und/oder plattenförmige Werkstücke ausgebildet sind.

10. Verfahren nach dem vorhergehenden Anspruch, **dadurch gekennzeichnet, dass** mit in mindestens einem Laminat ausgebildeten Öffnung eine elektrisch leitende Durchkontaktierung, die mit in die Öffnung eingefülltem elektrisch leitenden Werkstoff gebildet ist, ausgebildet wird oder in diese Öffnung ein elastisch verformbares elektrisch leitendes Element eingesetzt wird.

11. Verfahren nach einem der beiden vorhergehenden Ansprüche, **dadurch gekennzeichnet, dass** bei der thermischen Behandlung bevorzugt mit Druckkrafteinwirkung eine zumindest teilweise Sinterung des die mindestens eine elektrische Leiterbahn und/oder die mindestens eine elektrische Durchkontaktierung bildenden Werkstoffs erreicht wird.

## Claims

1. A microfluidic system for culturing or conducting analyses on living cells or biomolecules, in which a plurality of laminates (1.1, 1.2, ..., 1.x), which are arranged on top of one another, made of a polymer material and designed as films and/or panel-shaped workpieces, are joined to one another in a fluid-tight and integral manner, and at least one opening or at least one cut-out is formed in at least one of the laminates (1.1, 1.2, ..., 1.x) by which a channel (2) or a reservoir for receiving living cells or biomolecules is formed, and
at least one further opening (3) is formed in at least one of the laminates (1.1, 1.2, ..., 1.x), into which an actuator and/or a sensor (4) made of a ceramic or semiconducting material, or a carrier for an actuator or a sensor which is made of a ceramic or semiconducting material, is inserted, and at least one electrical strip conductor (6), by way of which electrical contacting of the actuator and/or sensor (4) can be achieved, is printed on the surface of the laminate (1.2) into which the actuator and/or sensor (4) or the carrier is inserted, or on the surface of a laminate (1.3) directly adjoining this laminate (1.2), the at least one actuator and/or sensor (4) being in touching contact with a liquid for the culturing of cells, a liquid in which biomolecules are contained or the cells or biomolecules contained in a channel or reservoir.

2. The system according to claim 1, **characterized in that** at least one electrical plated through-hole, in which an electrically conducting material is contained in an opening, and/or an elastically deformable, electrically conducting contact element is inserted in at least one of the laminates (1.1., 1.2, ..., 1.x).

3. A system according to any one of the preceding claims, **characterized in that** a flexibly deformable printed circuit board is arranged between two laminates that are arranged on top of one another and is connected to the laminates in a fluid-tight manner.

4. A system according to any one of the preceding claims, **characterized in that** a sensor (4), arranged within the stack formed by way of the laminates (1.1, 1.2, ..., 1.x), for detecting physical variables, in particular the temperature, the flow, the volume flow rate, the pressure, electromagnetic radiation, the electrical conductivity or the material composition, in particular the oxygen content, glucose content, lactate content, and/or an actuator, in particular a flow source, an electromagnetic radiation-emitting element, an electrode, an actively deformable membrane, a piezo actuator, a gas-permeable membrane, a temperature control element is provided.

5. A system according to any one of the preceding claims, **characterized in that** at least one permeable membrane, preferably a gas-permeable membrane, is arranged in the region of a channel (2) or reservoir.

6. A system according to any one of the preceding claims, **characterized in that** a channel (2) or reservoir is connected to a supply and/or removal device for liquid containing living cells and/or biomolecules.

7. A system according to any one of the preceding claims, **characterized in that** electrical and/or electronic elements, in particular an electrical resistor, a thermocouple, an inductive electrical element, a capacitive electrical element or active components, such as a transistor, are formed on at least one surface of at least one laminate by means of a printing method.

8. A system according to any one of the preceding claims, **characterized in that** a channel (2) or a reservoir is at least partially covered by a semi-permeable membrane (12) on a laminate (1.12)
or
a channel (2) or a reservoir is at least regionally covered by an elastically deformable membrane (13), and the membrane (13) is imprinted with electrically conducting printed structures 5.1, in particular strain gauges.

9. A method for producing a system according to any one of the preceding claims, **characterized in that** at least one opening or recess for forming a channel (2) or reservoir is formed in at least one laminate that is to be arranged between a laminate to be arranged above and one to be arranged beneath, and
at least one further opening for receiving an actuator and/or a sensor (4) made of a ceramic or semiconducting material, or a carrier of an actuator and/or of a sensor, which is made of a ceramic or semiconducting material, is formed in at least one laminate to be arranged above or beneath this laminate, and
an actuator and/or a sensor (4) or a carrier are inserted into the at least one further opening, and
at least one electrical strip conductor (6) for electrically contacting the actuator and/or sensor (4) is formed by way of a printing method on a surface of this laminate or of a laminate to be arranged directly adjacent thereto, and
the laminates are arranged stacked on top of one another such that the at least one opening forming a channel (2) or a reservoir and the at least one actuator and/or sensor (4) are positioned facing one another, and
the laminates forming the stack are connected to one another in a fluid-tight and integral manner by way of thermal treatment and simultaneously acting pressure forces, wherein laminates (1.1, 1.2, ..., 1.x) are used which are designed as films and/or panel-shaped workpieces.

10. The method according to the preceding claim, **characterized in that** an electrically conducting plated through-hole, which is formed by electrically conducting material added into the opening, is formed by way of opening formed in at least one laminate, or an elastically deformable, electrically conducting element is inserted into this opening.

11. The method according to any one of the two preceding claims, **characterized in that** at least partial sintering of the material forming the at least one electrical strip conductor and/or the at least one electrical plated through-hole is achieved during the thermal treatment, preferably by way of the action of pressure forces.

## Revendications

1. Système microfluidique pour la culture de ou l'analyse sur des cellules vivantes ou biomolécules, pour lequel plusieurs stratifiés (1.1,1.2, ...1.x) superposés composés d'un matériau polymère, qui sont réalisés sous la forme de films et/ou pièces en forme de plaque, sont reliés les uns aux autres de manière étanche au fluide et par liaison de matières et au moins une ouverture ou au moins une découpe est réalisée dans au moins un des stratifiés (1.1,1.2, ...1.x), avec laquelle est réalisé un canal (2) ou un réservoir pour la réception de cellules vivantes ou biomolécules, et
au moins une autre ouverture (3) est réalisée dans au moins un des stratifiés (1.1,1.2, ...1.x), dans laquelle est inséré un actionneur et/ou capteur (4) formé d'un matériau céramique ou semi-conducteur ou un support formé d'un matériau céramique ou semi-conducteur pour un actionneur ou capteur, et au moins un tracé conducteur électrique (6) est imprimé sur la surface du stratifié (1.2) dans lequel l'actionneur et/ou le capteur (4) ou le support est inséré ou sur la surface d'un stratifié (1.3) directement adjacent à ce stratifié (1.2), avec lequel une mise en contact électrique de l'actionneur et/ou du capteur (4) peut être obtenue ; dans lequel le au moins un actionneur et/ou capteur (4) est en contact avec un liquide pour la culture de cellules, liquide dans lequel des biomolécules sont contenues ou les cellules ou biomolécules contenues dans un canal ou réservoir.

2. Système selon la revendication 1, **caractérisé en ce que** dans au moins un des stratifiés (1.1,1.2, ...1.x) au moins un trou d'interconnexion électrique, pour lequel un matériau électroconducteur est contenu dans une ouverture et/ou un élément de contact électroconducteur déformable élastiquement est inséré.

3. Système selon l'une quelconque des revendications précédentes, **caractérisé en ce qu'**une carte de circuits imprimés déformable de manière flexible est disposée entre deux stratifiés superposés et reliée aux stratifiés de manière étanche au fluide.

4. Système selon l'une quelconque des revendications précédentes, **caractérisé en ce qu'**un capteur (4) disposé à l'intérieur de la pile formée avec les stratifiés (1.1,1.2, ...1.x) pour la détection de grandeurs physiques, en particulier de la température, du flux, du débit volumétrique, de la pression, d'un rayonnement électromagnétique, de la conductivité électrique ou de la composition de matière, en particulier de la teneur en oxygène, teneur en glucose, teneur en lactate et/ou un actionneur, en particulier une source de flux, un élément émettant un rayonnement électromagnétique, une électrode, une membrane déformable de manière active, un actionneur piézoélectrique, une membrane perméable aux gaz, un élément d'équilibrage de température, est disposé.

5. Système selon l'une quelconque des revendications précédentes, **caractérisé en ce qu'**au moins une membrane perméable, de préférence une membrane perméable au gaz, est disposée dans la zone d'un canal (2) ou réservoir.

6. Système selon l'une quelconque des revendications précédentes, **caractérisé en ce qu'**un canal (2) ou réservoir est relié à une amenée et/ou évacuation pour un liquide contenant des cellules vivantes et/ou biomolécules.

7. Système selon l'une quelconque des revendications précédentes, **caractérisé en ce que** des éléments électriques et/ou électroniques, en particulier une résistance électrique, un thermoélément, un élément électrique inductif, un élément électrique capacitif ou des composants actifs, tels qu'un transistor est réalisé au moyen d'un procédé d'impression sur au moins une surface d'au moins un stratifié.

8. Système selon l'une quelconque des revendications précédentes, **caractérisé en ce qu'**un canal (2) ou un réservoir est recouvert au moins par endroits avec une membrane semi-perméable (12) sur un stratifié (1.12)
ou
un canal (2) ou un réservoir est recouvert au moins par endroits avec une membrane élastiquement déformable (13) et la membrane (13) est imprimée avec des structures imprimées électroconductrices 5.1, en particulier des jauges de contraintes.

9. Procédé pour la fabrication d'un système selon l'une quelconque des revendications précédentes, **caractérisé en ce qu'**au moins une ouverture ou un évidement pour la réalisation d'un canal (2) ou réservoir est réalisé(e) dans au moins un stratifié, qui vise à être disposé entre un stratifié à disposer au-dessus et au-dessous, et
au moins une autre ouverture pour la réception d'un actionneur et/ou capteur (4) formé d'un matériau céramique ou semi-conducteur ou un support d'un actionneur et/ou capteur, qui est formé d'un matériau céramique ou semi-conducteur, est réalisé(e) dans au moins un stratifié à disposer au-dessus ou au-dessous de ce stratifié, et
un actionneur et/ou capteur (4) ou un support est inséré dans la au moins une autre ouverture et
au moins un tracé conducteur électrique (6) pour une mise en contact électrique de l'actionneur et/ou du capteur (4) est réalisé avec un procédé d'impression sur une surface de ce stratifié ou d'un stratifié à disposer directement au voisinage et
les stratifiés sont disposés de manière empilée les uns au-dessus des autres, de sorte que la au moins une ouverture formant un canal (2) ou un réservoir et le au moins un actionneur et/ou capteur (4) sont positionnés l'une par rapport à l'autre et
les stratifiés formant la pile sont reliés les uns aux autres de manière étanche au fluide et par liaison de matière au moyen d'un traitement thermique et de forces de pression agissant simultanément ; dans lequel les stratifiés (1.1,1.2, ...1.x), qui sont réalisés sous la forme de films et/ou de pièces en forme de plaques, sont insérés.

10. Procédé selon la revendication précédente, **caractérisé en ce qu'**un trou d'interconnexion électroconducteur, qui est formé avec un matériau électroconducteur versé dans l'ouverture, est réalisé avec une ouverture réalisée dans au moins un stratifié, ou un élément électroconducteur élastiquement déformable est inséré dans cette ouverture.

11. Procédé selon l'une des deux revendications précédentes, **caractérisé en ce que** pour le traitement thermique, de préférence avec une action de force de pression, un frittage au moins partiel du matériau formant le au moins un tracé conducteur électrique et/ou le au moins un trou d'interconnexion électrique est atteint.
